# EUROPÄISCHE PATENTANMELDUNG

(11) **EP 1 961 727 A1**
(43) Veröffentlichungstag der Anmeldung: **27.08.2008**
(21) Anmeldenummer: 07003860.9
(22) Anmeldetag: 26.02.2007
(51) Int. Cl.: C07C 45/46, C07C 49/84

(54) **Verfahren zur Herstellung von 2,4-Dihydroxyphenyl-4-methoxybenzyl-ketonen**

(71) Anmelder: Bayer CropScience AG, 40789 Monheim (DE)
(72) Erfinder: Die Erfindernennung liegt noch nicht vor

(57) **Zusammenfassung**

Die Erfindung betrifft ein Verfahren zur Herstellung von 2,4-Dihydroxyphenyl-4-methoxybenzyl Ketonen der Formel (I) durch Friedel Crafts Acylierung in Fluorwasserstoff (HF).

2,4-Dihydroxyphenyl-4-methoxybenzyl Ketone der Formel (I), in welchen
R¹ und R²
für Wasserstoff, Chlor, Fluor, Brom, lod, CF₃, Methyl, gegebenenfalls substituiertes Alkoxy, OCF₃, -C(CH₃)₃, -CH₂(CH₃)₂, -CH(CH₃)₂ stehen.
R³
für Wasserstoff, Cl, F, Br, gegebenenfalls substituiertes Alkyl, gegebenenfalls substituiertes Alkoxy, -C(CH₃)₃ steht und
X
für Hydroxy, F, Cl, gegebenenfalls substituiertes Alkoxy steht,

werden durch Umsetzung von Phenylessigsäurederivaten der Formel (II) mit Phenolcn der Formel (III) in flüssigem Fluorwasserstoff (HF) in hoher Ausbeute und hoher Reinhcit erzeugt.

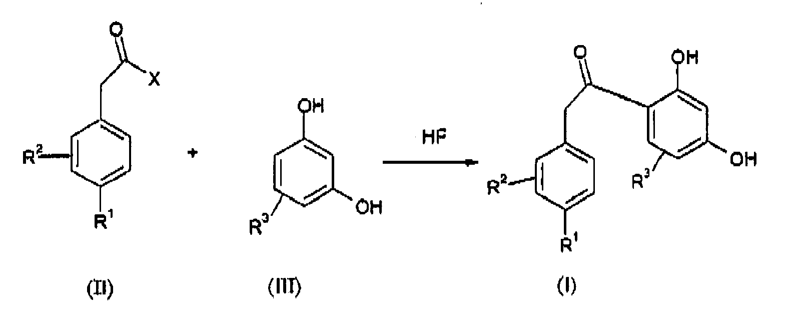

## Beschreibung

Die Erfindung betrifft ein. Verfahren zur Herstellung von 2,4-Dihydroxyphenyl-4-methoxybenzylketonen der Formel (I) durch Friedel Crafts Acylierung in Fluorwasserstoff (HF).

2,4-Dihydroxyphenyl-4-methoxybenzyl-ketone der allgemeinen Formel (I) sind wichtige Synthesebausteine zur Herstellung von Isoflavonen, wie beispielsweise dem Formononetin oder Genistein, Daidzein und Cournestrol.

2,4-Dydroxyphenyl-4-methoxybenzyl-ketone wurden beispielsweise mit sehr mäßiger Ausbeute durch die zeitaufwendige Hoeben-Hoesch Reaktion ausgehend von Phenylessigsäurenitril, Resoreinol und Chlorwasserstoff erhalten (W. Baker et al J.Ghem.Soc 1929, 2902),

Ferner wurde die Umsetzung von Resorcinol und Phenylessigsäureanhydrid oder der freien Säure in Gegenwart von Borontrifluoridetherat mit einer Ausbeute von 67% beschrieben (S-Mohaty et al., Current Science, May 20, 1988, vol. 57, N. 10 und US 5,981,775).

Des Weiteren wurde eine Synthese von Deoxybenzoin aus Resorcinol und 4-Methoxyphenylessigsäure in Gegenwart von 40 Moläquivalenten eines BF₃/Et₂O Komplexes beschrieben (T.A. Hase in J.Chem Soc, Perkin Trans. 1, 1991, 3005).

Außerdem wurden die Synthese von 2-Phenylacetophenonderivaten aus Phenol und Phenylessigsäure unter Verwendung von Dicyclohexylcarbodiimid (DCC) und 4-Dimethylaminopyridin (DMAP) beschriebe (WO 2005/054169).

Es ist weiterhin bekannt, dass Benzoine mittels Friedel Crafts Acylierung in Gegenwart von AlCl₃ erzeugt werden können (Indian J.Chem. vol, 6, 1968, p. 482).

Weiterhin ist bekannt, dass Polyhydroxybenzoine durch eine Mikrowellensynthese ionischer Flüssigkeiten in Gegenwart von Bis(trifluoromethly)sulfonyl-amin oder BF₃/OEt₂ zugänglich sind (Tetrahedron Letters, 47, 2006, 8375).

All diese Methoden weisen eine Reihe von Nachteilen auf Diese sind eine niedrige Ausbeute, lange Reaktionszeiten oder die Verwendung teurer Reagenzien wie DCC und DMAP. Die Anwendung von Katalysatoren wie BF₃ und AlCl₃ erzeugt große Mengen aufwändig zu entsorgender Abwässer. Der BF₃/Et₂O Komplex ist für eine großtechnische Synthese sogar praktisch ungeeignet, da er einen sehr niedrigen Flammpunkt besitzt.

Es wurde nun gefunden, dass die Synthese von 2,4-Dihydroxyphenyl-4-methaxybenzyl Ketonen der Formel (1), 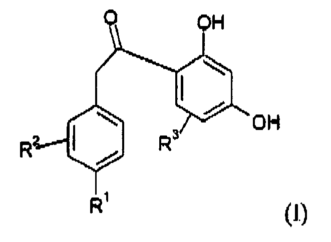 in welchen
- R¹ und R²: für Wasserstoff, Chlor, Fluor. Brom, Iod, CF₃, Methyl, Methoxy, gegebenenfalls substituiertes Alkoxy, -OCF₃, -C(CH3)₃, -CH₂(CH₃)₂, -CH(CH₃)₂ stehen,
- R³: für Wasserstoff, C1, F, Br oder gegebenenfalls substituiertes Alkyl, gegebenenfalls substituiertes Alkoxy, -C(CH3)₃ stehen und
- X: für Hydroxy, F, Cl oder gegebenenfalls substituiertes Alkoxy stehen,
durch Umsetzung von Phenylessigsäurederivaten der Formel (II) mit Phenolen der Formel (III) in flüssigem Fluorwasserstoff. (HF) mit hoher Ausbeute und in hoher Reinheit mögl ich ist.

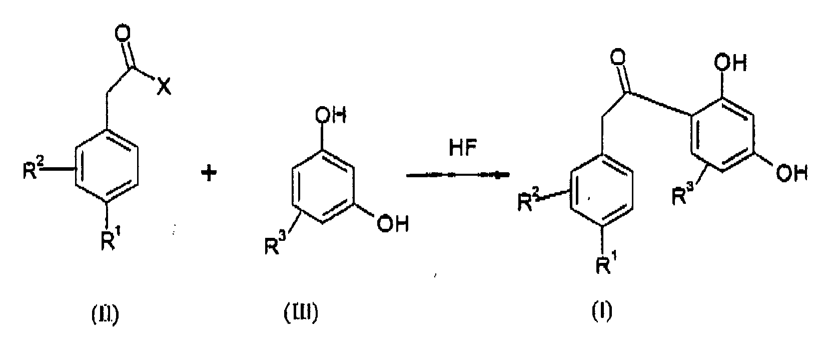

Ein weiterer Vorteil des Verfahrens besteht darin, dass HF mit einem Siedepunkt von 20°C leicht vom Produkt durch Destillation abgetrennt und daher vollständig zurückgeführt werden kann. Außerdem ist HF ein sehr preiswerter Rohstoff and wird großtechnisch im Tausend-Tonnen-Maßstab hergestellt und eingesetzt.
- R¹ und R²: stehen bevorzugt für Wasserstoff, Methyl, Methoxy, C₁-C₆-Alkoxy, -OCF₃, -C(CH₃)₃, -CH₂(CH₃)_{2,} -CH(CH₃)₂, oder Chlor, Fluor, Brom, Iod, CF₃,
- R¹ und R²: stehen besonders bevorzugt für Wasserstoff, Methyl, Methoxy, C₁-C₄-Alkoxy, -C(CH₃)₃, -CH₂(CH₃)₂, -CH(CH₃)₂, oder Chlor, Fluor, Brom, Iod, -CF₃.
- R¹: steht ganz besonders bevorzugt für Methoxy.
- R²: steht ganz besonders bevorzugt für Wasserstoff.
- R³: steht bevorzugt für Wasserstoff, C₁-C₆-Alkyl, Chlor, Fluor, Brom, C₁-C₆-Alkoxy, -C(CH₃)₃.
- R³: steht besonders bevorzugt für Wasserstoff, C₁-C₄-Alkyl, Cl, F, Br, C₁-C₄-Alkoxy, -C(CH₃)₃.
- R³: steht ganz besonders bevorzugt für Wasserstoff,
- X: steht bevorzugt für Hydroxy, Fluor, Chlor, C₁-C₆-Alkoxy und besonders bevorzugt für Hydroxy, Fluor, Chlor, C₁-C₄-Alkoxy.

Mögliche Substituenten für Alkyl und Alkoxy, sind: Fluor, Chlor, Brom. Iod, NO₂, CN, SCN, NCO.

Die Reaktion kann gegebenenfalls durch den Zusatz weiterer Katalysatoren beschleunigt werden. Beispielsweise können Katalysatoren wie z.B, Lewis Säuren wie BF₃, SbF₅, PF₅, BiF₃, AsF₃, AlCl₃, SbCl₅, TiCl₄, NbCl₅, SnCl₄ , SiCl₄ und InCl₃ eingesetzt werden. Bevorzugte Kutulysatoren sind; BF₃, SbCl₅, AlCl₃, SiCl₄, PF₅. Besonders bevorzugte Katalysatoren sind BF₃, AlCl₃, SbCl₅.

Die Reaktionstemperaturen können bei der Durchführung des erfindungsgemäßen Verfahrens in einem größeren Bereich variiert werden. Im Allgemeinen liegen die Temperaturen zwischen -10°C und 120°C. Bevorzugt sind Temperaturen zwischen 0°C und 50°C. Besonders bevorzugt sind Reaktionstemperaturen zwischen 20°C und 40°C.

Die Molverhältnisse des Fluorwasserstoffs zum Phenol der Formel (III) sind in einem weiten Bereich variierbar. Allgemein wird das erfindungsgemäße Verfahren mit Molverhältnissen von Fluorwasserstoff zum Phenol der Formel (III) zwischen 1:1 bis 1.00:l I durchgeführt. Bevorzugt sind Molverhältnisse von 50:1 bis 10:1.

Gegebenenfalls kann das erfindungsgemäße Verfahren in Anwesenheit weiterer Verdünnungsmittel durchgeführt werden. Als solche sind beispielsweise Ether, Freon, Dichlormethan, Dichlorethan, Tolual und Chlorbenzol geeignet.
Bevorzugt wird das Verfahren ohne weitere Verdünnungsmittel durchgeführt

Die Reaktion kann drucklos unter Eigendruck oder unter dem Druck eines Schutzgases durchgeführt werden.

### Herstellungsbeispiele

Herstellung von 1-(2,4-dihydroxyphenyl)-2-(4-methoxyphenyl)-ethanon

### Beispiel, 1

4-Methoxyphenylessigsäure (83.9 g), Resorcinol (55 g) und Fluorwasserstoff (450 g) werden bei 10°C im Autoklav vorgelegt und das Gemisch 12 h bei 20°C gerührt. Anschließend wird der Fluorwasserstoff bei 40°C abgedampft und der Niederschlag mit Wasser gewaschen und getrocknet. Man erhält 123 g (91 % der Theorie) des Produktes mit einer Reinheit von 96 % und einem Schmelzpunkt (Smp) 160-162°C.

### Beispiel 2

4-Methoxyphenylessigsaurechlorid (93 g), Resorcinol (55 g) und Fluorwasserstoff (450 g) werden bei -10°C im Autoklav vorgelegt und das Gemisch 12 h bei 20°C gerührt. Anschließend wird der Fluorwasserstoff bei 40°C abgedampft und der Niederschlag mit Wasser gewaschen und getrocknet. Man erhält 125 g (92 % der Theorie) des Produktes mit einer Reinheit von 96 %.

### Beispiel 3

4-Methoxyphenylessigsäure (83.9 g), Resorcinol (55 g) und Fluorwasserstoff (300 g) werden bei - 10°C im Autoklav vorgelegt und das Gemisch 12 h bei 20°C gerührt. Anschließend wird der Fluorwasserstoff bei 40°C abgedampft und der Niederschlag mit Wasser gewaschen und getrocknet Man erhält 120 g (89 % der Theorie) des Produktes mit einer Reinheit von 93 %.

## Patentansprüche

1. Verfahren zur Herstellung von 2,4-Dihydroxypheny-4-methoxybenzy-ketonen der Formel (1), 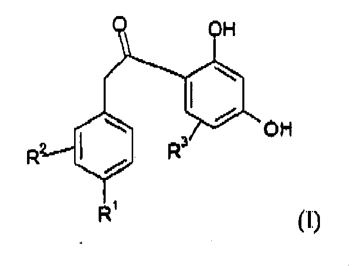 in welchen
R¹ und R² für Wasserstoff, Chlor, Fluor, Brom, Iod, CF₃, Methyl, gegebenenfalls scibstituiertes Alkoxy, -OCF₃, -C(CH₃)₃, -CH₂(CH₃)₂, -CH(CH₃)₂ stehen,
R³ für Wasserstoff, Cl, F, Br, gegebenenfalls substituiertes Alkyl, gegebenenfalls substituiertes Alkoxy, -C(CH₃)₃ steht und
X für Hydroxy, F, Cl, gegebenenfalls substituiertes Alkoxy steht,
durch Umsetzung von Phenylessigsäurederivaten der Formel (II) mit Phenolen der Formel (III) mit Fluorwasserstoff.

2. Verfahren gemäß Anspruch 1, wobei
R¹ und R² für Wasserstoff, Chlor, Fluor, Brom, Iod, CF₃, Methyl, C₁-C₆-Alkoxy, -OCF₃, -C(CH₃)₃, -CH₃(CH₃)₂, -CH(CH₃)₂ stehen,
R³ für Wasserstoff, Cl, F, Br, C₁-C₆-Alkyl, C₁-C₆-Alkoxy, -C(CH₃)₃ steht und
X für Hydroxy, F, Cl, C₁-C₆-alkoxy steht.

3. Vertähren genie Anspruch 1, wobei
R¹ und R² für Wasserstoff, Chlor, Fluor, Brom, lod, CF₃, Methyl, C₁-C₄-Alkoxy, -C(CH₃)₃, -CH₂(CH₃)₂ -CH(CH₃)₂ stehen,
R³ für Wasserstoff. Cl, F, Br, C₁-C₆-Alkyl, C₁-C₄-Alkoxy, -C(CH₃)₃, steht und
X für Hydroxy, F, Cl, C₁-C₄-Alkoxy steht.

4. Verfahren zur Herstellung von 1-(2,4-dihydroxyphenyl)-2-(4-methoxyphenyl)-ethanon, **dadurch gekennzeichnet, dass** 4-Methoxyphenylessigsäure oder 4-Methoxyphenylessigsaurechlorid mit Resorcinol und mit Fluorwasserstoff umgesetzt wird.

5. Verfahren gemäß Anspruch 1. **dadurch gekennzeichnet, dass** das Molverhältnis von Fluorwasserstoff zum Phenol der Formel (III) im Bereich von 50; 1 bis 10:1 liegt.

6. Verfahren gemäß Anspruch 1, **dadurch gekennzeichnet, dass** die Reaktionstemperatur zwischen 10°C und 50°C liegt.

7. Verfahren gemäß Anspruch 1, **dadurch gekennzeichnet, dass** die Reaktion in Anwesenheit einer Lewis Säure abläuft,

8. Verfahren zur Herstellung von 1-(2,4-dihydroxyphenyl)-2-(4-methoxyphenyl)-ethanon, **dadurch gekennzeichnet, dass** 4-Methoxyphenylessigsäure oder 4-Methoxyphenylessigsäurechlorid mit Resorcinol und mit Fluorwasserstoff, bei einem Molverhältnis von Fluorwasserstoff zu Resorcinol im Bereich von 50:1 bis 10:1 und bei einer Temperatur in einem Bereich von 0°C bis 40°C, umgesetzt wird.
